# EUROPÄISCHE PATENTSCHRIFT

(11) **EP 2 465 918 B1**
(45) Veröffentlichungstag und Bekanntmachung des Hinweises auf die Patenterteilung: **20.11.2013**
(21) Anmeldenummer: 11002907.1
(22) Anmeldetag: 07.04.2011
(51) Int. Cl.: C12M 1/16, C12M 1/26

(54) **Biomassetrockenfermenter mit kontinuierlicher Beschickung und Entnahme**
Biomass dry fermenter with continuous loading and emptying
Fermenteur sec de biomasse doté d'une alimentation et d'une extraction en continu

(30) Priorität: 18.12.2010 EP 10015806; 26.01.2011 EP 11000606
(43) Veröffentlichungstag der Anmeldung: 20.06.2012
(73) Patentinhaber: Hoffmann, Gerhard, 97258 Hemmersheim (DE)
(72) Erfinder: Hoffmann, Gerhard, 97258 Hemmersheim (DE)
(74) Vertreter: Pöhner, Wilfried Anton

(56) Entgegenhaltungen:
- EP-A1- 0 241 357
- EP-A1- 2 020 434
- WO-A1-2006/089766
- WO-A1-2011/076444
- DE-A1-102006 024 081
- DE-A1-102007 014 417
- DE-A1-102007 025 903
- DE-A1-102008 038 262

## Beschreibung

Die Erfindung bezieht sich auf einen Biomassetrockenfermenter mit kontinuierlicher Beschickung von Biomassesubstrat und kontinuierlicher Entnahme des vergorenem Biomassesubstrates, gemäß Oberbegriff des Anspruches 1.

Ein Fermenter ist ein Teil einer Biogasanlage, in der aus einem Biomassesubstrat Gas gewonnen wird, dass zur Energiegewinnung genutzt wird. Zumeist wird es in einem Blockheizkraftwerk in einem Verbrennungsmotor mit angekuppeltem Generator in Wärme und in elektrische Energie umgewandelt. Der entscheidende Vorzug dieses Verfahrens ist, dass es CO² - neutral ist, wenn man von den Aufwendungen zum Einbringen der Biomasse und beim Betrieb der Gasanlage absieht. Letztendlich ist es also eine Nutzbarmachung solarer Energie. Als Biomasse sind u.a. die verschiedensten Feldfrüchte wie Gras und Getreide sowie pflanzliche Abfälle und tierische Extremente geeignet.

Für die Umwandlung dieser Biomasse in ein Gas - vorwiegend Methangas - werden zahlreiche Mikroben tätig, die im wesentlichen in den folgenden vier Phasen wirken, nämlich
- der Hydrolyse,
- der Acidogenese oder Versäuerungsphase,
- der Acetogenese oder Essigbildenden Phase sowie
- als letzter Phase in der Methanophase oder Methanbildenden Phase.

Die ersten drei Phasen benötigen für eine optimale Wirkung jeweils etwa gleiche Umgebungsbedingungen, nämlich 25 bis 35 Grad Temperatur und einen pH-Wert zwischen 4,5 und 6,5. Diese Werte unterscheiden sich deutlich von der letzten und vierten Phase, der Methanbildenden Phase. Deshalb ist es sinnvoll, die ersten drei Phasen in einem gemeinsamen Reaktor, einem Fermenter ablaufen zu lassen und das aus diesen Prozessen entstehende Zwischenprodukt, dass sog. "Perkolat" in einen weiteren Reaktor zu überführen, in dem dann in leicht alkalischer Umgebung das Perkolat zu einem großen Teil in Methangas umgewandelt wird.

Insbesondere zur Fermentation von Gras, Stroh und anderen faserigen oder größer formatigen Feldprodukten ist das sog. Trockenfermentationsverfahren geeignet. Dabei ist eine Zerkleinerung des Feldproduktes von Vorteil, das mit einem Perkolat oder einem anderen flüssigen Stoff, wie z. B. Gülle, geimpft wird, sodass die Vergärung einsetzt. Nach Abschluss der Gärung wird das vergorene Biomassesubstrat in Perkolat und Feststoffe getrennt. Die Feststoffe werden als Dünger eingesetzt und das Perkolat in einer letzten Reaktorstufe zum größten Teil in Methangas umgewandelt.

Auf aktuellem Stand der Technik ist es üblich, dass die einzelnen "Batches" in gasdichte Behälter von der Größe einer Kraftfahrzeuggarage eingebracht, geimpft und erwärmt und nach Abschluss der Versäuerungsphase wieder entnommen werden. Dieses Verfahren hat den Nachteil, dass eine Biogasanlage stets mehrere solcher "Garagen" benötigt, von denen grundsätzlich nur ein Teil Perkolat erzeugt, während weitere "Garagen" beladen oder aufgeheizt oder entladen werden müssen.

Ein weiterer Nachteil ist, dass zur Trennung von Perkolat und Feststoffen zumeist Spaltenböden eingesetzt werden, durch welche das Perkolat hindurch läuft. Die Spalten neigen jedoch zur Verstopfung, woraufhin der Prozess unterbrochen werden muss, Sauerstoff in den Fermenterbehälter eintritt, dass bereits angegorene Biomassesubstrat zur Seite geschoben werden muss und die Spalten gesäubert werden müssen.

Um diese Schwierigkeiten zu umgehen, beschreibt die DE 10 2007 025 903 einen Fermentationsraum, in dem die Biomasse auf einer "Bewegungseinheit" horizontal verschoben wird. Die Bewegungseinheit kann eine bewegbare Plattform, ein Schubboden, ein Kratzboden oder ein Förderband sein.

Nachteilig ist, dass diese Bewegungseinheiten die gesamte Fläche des Fermentationsraumes umfassen. Deshalb lastet die gesamte Biomasse auf der Bewegungseinheit, sodass entsprechend hohe Kräfte zu ihrer Bewegung erforderlich sind. Ein weiterer Nachteil der großen Fläche ist, dass eventuelle Verstopfungen oder Verklemmungen auf der gesamten Fläche des Fermenters möglich sind, sodass eine Behebung der Verstopfungen und/oder der Austausch eines mechanischen Bauteils die Entleerung des gesamten Fermenters erfordert.

Nachteilig ist auch, dass diese Bewegungseinrichtungen eine sehr große Oberfläche haben und deshalb mit dieser sehr großen Fläche der Korrosion durch die Säuren des Fermenters ausgesetzt sind. Entsprechend umfangreich ist auch der Verschleiß und damit die Betriebskosten. Dadurch wird nicht nur die CO²-Bilanz verschlechtert, sondern auch der wirtschaftliche Erfolg der Biogasanlage geschmälert.

Aus der WO 2006/089766 ist ein Fermenterbehälter bekannt, dem kontinuierlich Biomassesubstrat zugeführt und nach der Verarbeitung entnommen wird, wobei die Anordnung nach außen zu nicht gasdicht abgeschlossen ist, sodass ein Sauerstoffaustausch möglich wird. Zur Unterstützung der Durchmischung ist ein horizontales Paddelrührwerk im Inneren des Fermenterbehälters angebracht und ebenso ist die Trennung des Biomassesubstrates in ein Perkolat und in die Feststoffe vorgesehen.

Auf diesem Hintergrund hat sich die Erfindung die Aufgabe gestellt, für einen Fermenterbehälter eine Anordnung zu entwickeln, die einen kontinuierlichen Durchfluss des Biomassesubstrates bei einer etwa gleichbleibenden Temperatur und unter Abschluss von Sauerstoff ermöglicht, wobei die zur Bewegung des Substrates dienenden mechanischen Vorrichtungen einfach, robust und kompakt sein sollen.

Als Lösung lehrt die Erfindung eine Vorrichtung mit den in Anspruch 1 eingegebenen Merkmalen.

Ein wesentliches Merkmal der Erfindung ist also, dass die - relativ hohe - Masse der Biomasse selbst zu ihrem Weitertransport durch den Fermenter eingesetzt wird, indem Sie von Ihrer Schwerkraft auf einer schrägen Fläche voran gedrückt wird. Es ist ein Verdienst der Erfindung, erkannt zu haben, dass dadurch die zur Bewegung der Biomasse erforderliche Arbeit aus dem Fermentationsraum heraus und in die zu deren Befüllung eingesetzte Vorrichtung - hier eine Schnecke - verlagert wird. In diesem Abschnitt des Prozesses noch vor der eigentlichen Versäuerung ist die Biomasse noch nicht versäuert und daher nicht so aggressiv gegen die Fördereinrichtung. Sie ist auch noch nicht mit dem Perkolat durchzogen und hat daher noch ein sehr viel geringeres, spezifisches Gewicht. Darüber hinaus ist die Struktur der Biomasse in aller Regel sehr klein- und vielteilig, sodass sie zumeist als ein Schüttgut in einer Förderschnecke mit einem relativ sehr vielen höheren Wirkungsgrad befördert werden kann als das innerhalb des Fermenters möglich ist.

Ein weiteres, wichtiges Merkmal der Erfindung ist das Paddelrührwerk, dessen Welle horizontal im Fermenterbehälter angeordnet ist. In der Praxis wird in der Regel die Welle und deren Durchlass durch die Seitenwände des Fermenterbehälters nicht von Biomasse beaufschlagt sein, sodass nur die Paddel in die Biomasse hineinragen. Dadurch ist die Welle frei von dem erhöhten Verschleiß, den der Kontakt mit der angesäuerten, aggressiven Biomasse bewirken würde.

Falls die Decke des Fermenterbehälters eine gasdichte Plane ist, kann diese zur Reparatur, zur Wartung oder zur Reinigung des Paddelrührwerkes abgeklappt werden und das Paddelrührwerk ohne eine Entnahme der Biomasse gewechselt oder gereinigt werden. Der Vorteil des Paddelrührwerkes ist also seine relativ sehr kleine Kontaktfläche mit dem aggressiven Biomassensubstrat.

In gleicher Weise präferiert die Erfindung auch für die Vorrichtung zur Entnahme eine sehr kompakte, robuste und leicht auswechselbare Vorrichtung, nämlich eine Schnecke. Schnecken zum Transport von flüssigen oder viskosen Stoffen sind schon seit Jahrtausenden bewährt. Da in der erfindungsgemäßen Annordnung das schlammige Biomassesubstrat auf eine Linie zusammengedrückt wird, hat die Erfindung vorgesehen, längs dieser Linie eine zweite Förderschnecke zur Entnahme des vergorenen Substrates anzuordnen. Im Vergleich zu anderen Bewegungsvorrichtungen mit erheblich größeren Abmessungen und erheblich größerer Oberfläche ist eine Schnecke vergleichsweise kostengünstig, durch ihre Bauform sehr robust und kann im Fall eines Wechsels sogar notfalls ohne eine Entleerung des Fermenterbehälters "herausgeschraubt" werden.

Wenn eine Entnahme des Biomassesubstrates extrem aufwendig ist, so kann eine neue Schnecke notfalls auch durch ihre Schraubenwirkung von der Seite her in des Substrat "eingeschraubt" werden, wenn der Kopf der Schnecke mit einer dafür geeigneten Schneide ähnlich wie bei einem Spiralbohrer versehen ist. Sowohl der Antrieb der zweiten Förderschnecke als auch der Motor für das Paddelrührwerkes können extern außerhalb des Fermenterbehälters angeordnet werden und durch eine Drehdichtung effizient gegen das aggressive Biomassesubstrat geschützt werden.

Es ist ein wesentliches Element der Erfindung, dass der Boden des Fermenterbehälters an drei Seiten geneigt ist. Diese Form entspricht im Prinzip einem vertikal halbierten Trichter, der an seinen Schnittflächen durch je eine ebene Platte erweitert ist. Dabei ist das erforderliche Gefälle in Abhängigkeit von den zu fermentierenden Biomassen und den Dimensionen des Fermenterbehälters sowie dem Haftreibungs- und Gleitreibungskoeffizient des Bodens vom Fermenterbehälter gegenüber der Biomasse zu wählen. Die Erfindung hat dafür Werte zwischen 1 Grad und 60 Grad vorgesehen.

Es ist eine vorteilhafte Ausführungsvariante, dass der Boden des Fermenterbehälters aus drei Ebenen besteht, die jeweils zur Mitte des Behälters hin abfallen und dann eine V-förmige Rille bilden. Es ist sinnvoll, die zweite Förderschnecke in diese V-förmige Rille hineinzusetzen, da sie dort die auf den schrägen Flächen hinabgeleitete Biomasse wirkungsvoll fassen und aus dem Fermenterbehälter hinausfördern kann.

Das Prinzip der Erfindung ist für jede Art der Beschaffenheit des Bodens und der Seitenwände des Fermenterbehälters geeignet. Wenn die Investitionskosten möglichst gering sein sollen, so ist auch ein Betrieb mit einer Oberfläche aus rohem Beton denkbar. Das Gefälle muss dann relativ groß sein. Wenn die Oberfläche mit einem Kunststoff beschichtet wird, ist der Haft- und der Gleitreibungseffizient sehr viel niedriger, sodass ein geringeres Gefälle erforderlich ist, woraus bei gleichem Grundriss ein höheres Arbeitsvolumen des Fermenters resultiert.

Besonders vorteilhaft ist eine Beschichtung des Bodens und/oder der Seitenwände mit Edelstahl. Zwar sind die primären Investitionen relativ hoch, aber die zu erwartende Lebensdauer relativ sehr hoch und der erreichbare Haft- und Gleitreibungskoeffizient besonders niedrig.

Deshalb kann sich der höhere Aufwand für eine Beplankung mit Edelstahl durch reduzierte Betriebskosten und reduzierten Aufwand bei Wartung, Reinigung und Instandhaltung sehr schnell wieder amortisieren und über die Lebensdauer der Anlage hinweg eine höhere Rendite ermöglichen.

Das Paddelrührwerk weist in einem einfachen Fall zahlreiche Paddel von gleicher Länge auf, die dann jeweils mit gleicher Tiefe in die Biomasse eintauchen. Wenn gewünscht wird, dass die Paddel bis in die Nähe der geneigten Bodenflächen des Fermenters herunter reichen, so müssen die Paddel auf den Außenseiten kürzer und in der Mitte des Rührwerkes relativ lang sein. Der Vorteil ist, dass dann die Durchmischung und weitere Förderung der Biomasse verstärkt wird. Wenn die Biomasse mittels der ersten Förderschnecke über den Rand des Fermenterbehälters hinweg eingefüllt wird, so bildet sich im Fermenterbehälter bei den meisten Arten von Biomasse ein Schüttkegel aus, der den Innenraum nicht vollständig ausnutzt. Wenn unterhalb des Auslasses der ersten Förderschnecke eine vorrangig horizontal ausgerichtete Verteilerschnecke angeordnet wird, kann diese Verteilerschnecke die eintreffende Biomasse auf die gesamte, wirksame Breite des Fermenterbehälters verteilen. Dadurch wirkt ein Fermenter noch effizienter und kann innerhalb einer gegebenen Zeit eine größere Menge von Biomasse versäuern oder kann eine vorgegebene Menge an Biomasse innerhalb einer kürzeren Zeit verarbeiten.

Zur Einleitung des Fermentationsprozesses ist es bekanntlich erforderlich, dass die Biomasse "geimpft" wird, dass also eine geringe Anzahl von Mikroben und Bakterien eingebracht wird, die sich dann dank der für sie vorteilhaften "Nahrung" im Fermenterbehälter sehr zahlreich vermehren.

Da die Durchlaufzeit der Biomasse durch einen erfindungsgemäßen Fermenterbehälter gegenüber bisherigen Verfahren erheblich verkürzt ist, haben die Bakterien nicht ausreichen Einwirkungszeit, um selbsttätig aus dem unteren Bereich des Fermenterbehälters in die oberen Bereiche hin zu der frischen Biomasse zu wandern. Deshalb wird im stationären Prozess stets ein Teil des gewonnenen Perkolats wieder zurück in den Fermenterbehälter geführt. Alternativ kann extern zugekauftes Perkolat, Gülle oder eine anderes Impfmaterial eingespeist werden.

Das Impfmaterial wird entweder in die erste Förderschnecke oder direkt in den Fermenterbehälter oder an die Verteilerschnecke geleitet. Die Zuleitung in den Fermenterbehälter weist sinnvoller Weise zahlreiche Verteildüsen auf, die wie Duschköpfe im Sanitärbereich das flüssige Perkolat über die Biomasse verteilen.

Wie erwähnt benötigt der Prozess eine Temperatur von 25 bis 35 Grad, weil nur in diesem Temperaturbereich die Mikroben optimal wirken. Diese Temperatur kann im Sommer auch in nördlichen Breiten ohne eine Zusatzheizung erzielt werden. Wenn die Anlage jedoch ganzjährig betrieben soll, schlägt die Erfindung vor, dass eine Heizung an den Seiten und/oder am Boden und/oder an der Decke des Fermenterbehälters angebracht wird. Bevorzugt sollten die dafür eingesetzten Heizelemente - wie z. B., Wasserrohre - nicht innerhalb des Fermenterbehälters sondern unterhalb dessen Oberfläche verlaufen. Eine sinnvolle Variante dafür ist es, z. B. Metallrohre an der Rückseite einer Edelstahlbeplankung großflächig zu befestigen.

Da der Fermenterbehälter gasdicht sein soll, ist es sinnvoll, ihn aus Beton, Stahlbeton oder Metallplatten zu erstellen. Die Decke des Fermenterbehälters kann dann ebenfalls aus Beton oder Metall bestehen. Eine Alternative ist eine gasdichte Plane, die wie bei einem Lastkraftwagen am Rand verzurrt und damit gasdicht ist, aber bei einer Havarie oder zur Wartung relativ schnell zur Seite geschlagen werden kann.

Die Erfindung empfiehlt, dass in die Seiten des Fermenterbehälters gasdichte Kontrollöffnungen eingebracht werden sollten, durch die Proben entnommen werden können. Wenn sie mit Glasfenstern ausgestattet werden, kann der Versäuerungsprozess auch visuell kontrolliert werden kann.

Insbesondere bei einer massiven Decke ist eine gasdichte Einstiegsluke und ein Überdruckventil sinnvoll.

Ein weiteres Verdienst der Erfindung ist es, die Erzeugung von Perkolat aus Biomassesubstrat nicht nur von der Methangasbildung zu trennen und in einen getrennten Prozess auszulagern, sondern den Prozess innerhalb des Fermenters kontinuierlich ablaufen zu lassen. Dazu wird das Biomassesubstrat in etwa kontinuierlich eintreffenden Portionen oder in einem etwa kontinuierlichen Strom in den Eingang des Fermenterbehälters eingebracht wird und mit Perkolat und/oder Gülle oder einem anderen Material geimpft wird, das zur Einleitung des Versäuerungsprozesses geeignete Mikroben enthält. Dann wird das geimpfte Biomassesubstrat innerhalb des Fermenterbehälters kontinuierlich weiter bewegt und dabei zu Perkolat umgewandelt.

Während dieser- recht langsamen - Bewegung des Substrates wird es unter Abschluss von Sauerstoff auf die geeignete Temperatur von etwa 25 bis 35 Grad gebracht, so dass die Hydrolyse, die Acidogenese oder Versäuerungsphase und die Acetogenese oder Essigbildenden Phase ablaufen. Am Ende ist auf der Entleerungsseite des Fermenterbehälters das Perkolat entnehmbar.

Das fertige Perkolat steht zur Überführung in einen weiteren Reaktor zur finalen Umwandlung in Biogas bereit. In der Praxis ist es sinnvoll, einen geringen Teil des Perkolats - zumeist etwa rund 20% - wieder zurück in den Fermenterbehälter zu führen, um damit das Biomassesubstrat zu impfen, so dass der Prozess der Versäuerung kontinuierlich weiter läuft.

Die entscheidenden Vorzüge des erfinderischen Verfahrens sind, dass während der Zeit bis zum Erreichen der für Mikroben erforderlichen "Betriebstemperatur" einer frischen Portion von Biomassesubstrat bereits von der übrigen Menge der im Fermenter befindlichen Biomasse Perkolat produziert wird. Es wird also über die gesamte Zeit hinweg etwa gleichmäßig verteilt Perkolat produziert.

Ein weiterer Vorteil ist, dass die Bewegung des Substrates innerhalb des Fermenters über die gesamte Betriebszeit hinweg gleichmäßig verteilt wird. Dadurch verteilt sich auch der Bedarf an mechanischer Energie für diese Bewegung auf die gesamte Betriebsdauer, so dass
- anders als bei den bisher bekannten, diskontinuierlichen Verfahren
- der erforderliche Spitzenwert des mechanischen Energie erheblich geringer ist, so dass die Investitionskosten und die Grundgebühren der Kosten des eigenen Energiebedarfes erheblich niedriger sind.

Im Folgenden sollen weitere Einzelheiten und Merkmale der Erfindung anhand eines Beispiels näher erläutert werden. Dieses soll die Erfindung jedoch nicht einschränken, sondern nur erläutern. Es zeigt in schematischer Darstellung:
Figur 1:
   Schnitt durch einen erfindungsgemäßen Biomassetrockenfermenter parallel zur ersten Förderschnecke 3 und senkrecht zur zweiten Förderschnecke 4
Figur 2
   Schnitt wie vor, jedoch horizontal, unterhalb der Decke
Figur 3
   Schnitt wie vor, jedoch längs und parallel zur zweiten Förderschnecke.

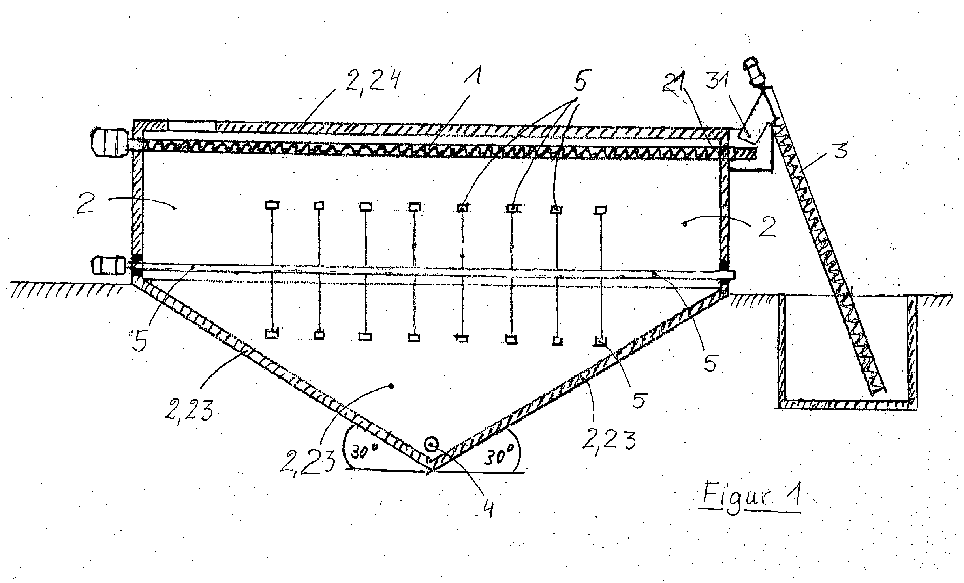

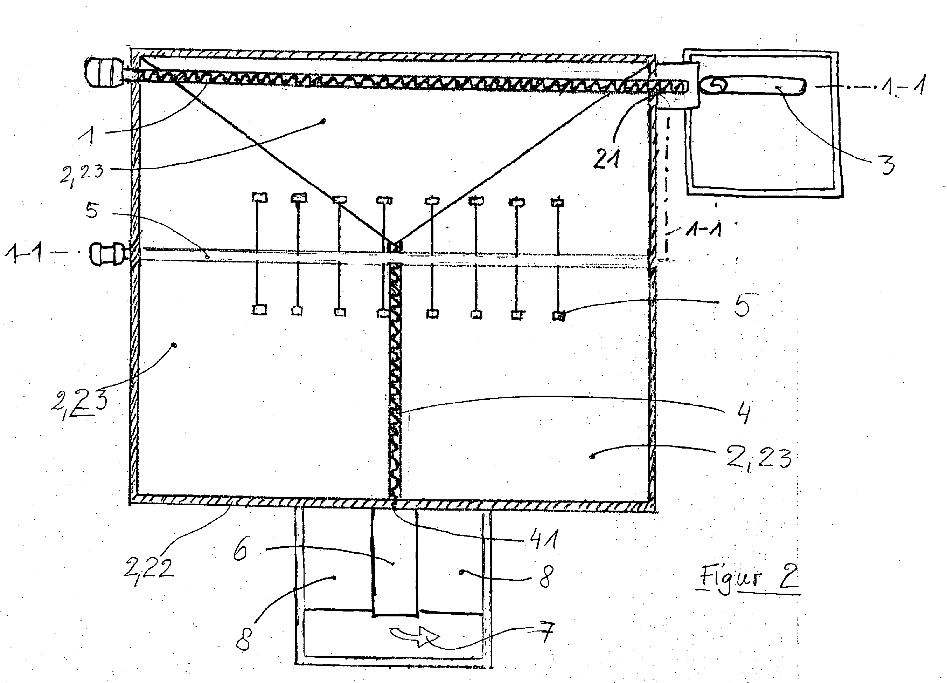

In **Figur 1** ist ein erfindungsgemäßer Biomassetrockenfermenter in zwei vertikalen Ebenen geschnitten. Die erste Schnittebene verläuft senkrecht zu der zweiten Förderschnecke 4 in der V-förmigen Rille zwischen den beiden zueinander geneigten Ebenen des Bodens 23 des Fermenterbehälters 2 und durch die Längsachse des Paddelrührwerkes 5. Die zweite Schnittebene verläuft parallel zur ersten, jedoch durch die Verteilerschnecke 1 und die erste Förderschnecke 3 hindurch, sodass diese beiden Schnecken im Schnitt dargestellt werden. Die beiden Schnittebenen sind in Figur 2 mit einer strichpunktierten Linie eingetragen und mit "1-1" bezeichnet.

In Figur 1 ist dank der Darstellung in zwei Schnittebenen die Bewegung des Biomassesubstrates durch den erfindungsgemäßen Biomassetrockenfermenter hindurch sehr gut nachvollziehbar: Dass Biomassesubstrat wird in die rechts eingezeichnete Grube abgeladen und durch die erste Förderschnecke 3 daraus heraus gefördert. Es steigt auf bis zum oberen Rand der Fermenterbehälters 2 und wird durch einen ersten, gasdichten Abschieber 31 hindurch zum Eingang 21 des Fermenterbehälters 2 gefördert.

In diesem Bereich wird es - in diesem Ausführungsbeispiel - von einer Verteilerschnecke 1 aufgenommen, die es über die gesamte Breite des Fermenterbehälters 2 verteilt. Aus der Verteilerschnecke 1 fällt das Substrat nach unten und gerät dadurch auf die zum Betrachter hin geneigte Bodenfläche 23 des Fermenterbehälters 2. Diese Bodenfläche 23 erscheint in Figur 1 als ein Dreieck. In Figur 1 ist gut nachvollziehbar, dass Biomassesubstrat auch auf den anderen beiden Bodenflächen 23 auftrifft und auch auf diesen bis zur Mitte des Fermenterbehälters 2 geleitet wird, sofern die Bodenflächen 23 ein ausreichendes Gefälle aufweisen.

Unten in der Mitte des Fermenterbehälters 2 ist die zweite Förderschnecke 4 im Schnitt nur mit Ihrer Antriebsachse und mit dem äußeren Mantelrohr erkennbar, denn sie weist senkrecht auf den Betrachter zu. Es ist gut nachvollziehbar, dass die Förderschnecke 4 das Biomassesubstrat im Fermenterbehälter 2 vollständig ergreifen und befördern kann. In der Perspektive von Figur 1 bewegt sie das Substrat auf den Betrachter zu.

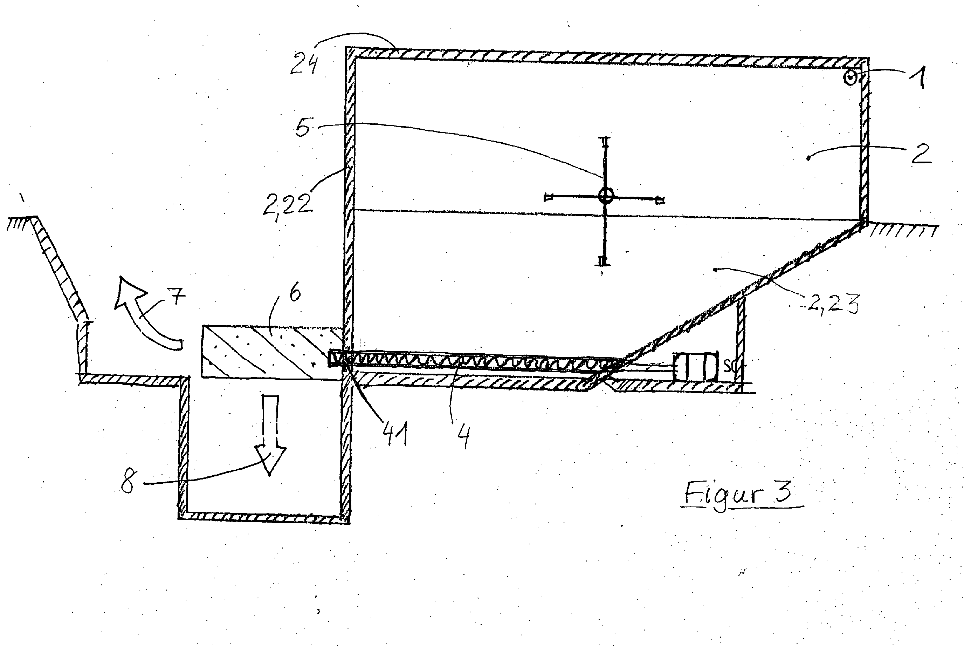

Figur 1 verdeutlicht ebenfalls sehr gut die Funktion des Paddelrührwerkes 5, auch wenn die Biomasse in Figur 1 nicht eingezeichnet ist: Wenn die Achse dieses Paddelrührwerkes 5 im Uhrzeigersinn rotiert, schwenken die Paddel in die darunter befindliche Biomasse hinein und bewegen sie - in Figur 1 in Richtung auf den Betrachter zu. Diese Wirkung des Paddelrührwerkes 5 kann auch in den beiden Figuren 2 und 3 nachvollzogen werden.

In **Figur 2** ist der in Figur 1 zeichnerisch senkrecht geschnittene Trockenfermenter nunmehr horizontal geschnitten und zwar direkt unterhalb seines Deckels 24, wodurch der Blick auf sein Inneres frei wird. Figur 2 zeigt rechts oben, wie aus dem Abwurfbehälter für das Biomassesubstrat die erste Förderschnecke 3 das Biomassesubstrat zum Eingang 21 des Fermenterbehälters und zur Verteilerschnecke 1 befördert. Dann bewegt die Verteilerschnecke 1 das Biomassesubstrat weiter in den Fermenterbehälter 2 hinein und verteilt es entlang dessen Breitseite. In Figur 2 ist gut nachvollziehbar, wie das Substrat auf der dreieckigen Bodenfläche 23 und den beiden angrenzenden trapezförmigen Bodenflächen 23 des Fermenterbehälters 2 nach unten gleitet, bis es auf die Förderschnecke 4 trifft, die es zur Entleerungsseite 22 des Fermenterbehälters 2 und durch den zweiten gasdichten Abschieber 41 hindurch in den Separator 6 befördert, wo es in das Perkolat 7 und die Feststoffe 8 aufgetrennt wird.

In Figur 2 sind die beiden Schnittebenen der Figur 1 mit einer strichpunktierten Linie eingetragen und mit "1-1" bezeichnet.

**Figur 3** zeigt einen weiterer Schnitt durch einen erfindungsgemäßen Biomassetrockenfermenter, wobei die Schnittebene senkrecht zu der Schnittebene der Figur 1 und durch die zweite Förderschnecke 4 und durch die tiefste Unterkante des Fermenterbehälters 2 hindurch verläuft.

In Figur 3 ist oben rechts die Verteilerschnecke 1 im Querschnitt zu sehen, die in dieser Perspektive nur Ihre Achse und ihr Mantelrohr zeigt. In Figur 3 wird gut nachvollziehbar, dass das von der Verteilerschnecke 1 geförderte Biomassesubstrat durch seine Schwerkraft nach unten auf die geneigte Bodenfläche 23 fällt, und - bei ausreichendem Gefälle b.z.w. durch einen Belag mit an das Gefälle angepasstem Gleitreibungskoeffizient - auf dieser geneigten Ebene nach unten rutscht. Dort wird das - mittlerweile vergorene - Biomassesubstrat von der zweiten Förderschnecke 4 erfasst und - in Figur 3 nach links - zur Entleerungseite 22 des Behälters 2 geführt. Dort tritt das Substrat durch den zweiten gasdichten Abschieber 41 in einen Separator 6 ein.

Die Funktion des Separators 6 ist in Figur 3 nicht dargestellt, sondern nur das Ergebnis seiner Tätigkeit, nämlich die Trennung des vergorenen Biomassesubstrates in Feststoffe 8, die in einen Behälter unterhalb des Separators 6 niederfallen und in das Perkolat 8, das vom Separator 6 weiter geleitet wird bis zur vierten Stufe der Biogasaufbereitung, dem Methangasreaktor.

In Figur 3 ist auch die Funktion des Paddelrührwerkes 5 gut nachzuvollziehen: Wenn man sich vorstellt, dass der Fermenterbehälter 2 mit dem - in Figur 3 nicht dargestellten - Biomassesubstrat fast bis zur Drehachse des Paddelrührwerkes 5 gefüllt ist und die Paddel des Paddelrührwerkes 5 im Uhrzeigersinn rotieren, dann tauchen sie in das Biomassesubstrat ein, rühren es dadurch um und verschieben es auch innerhalb des Fermenterbehälters 2 weiter zur Entleerungsseite 2 hin.

In diesem Bereich wird das Substrat, wie auch in Figur 1 ersichtlich ist, durch die V-förmig aufeinander zulaufenden Bodenflächen 23 des Fermenterbehälters 2 in dessen Mitte geschoben, und dort von der zweiten Förderschnecke 4 erfasst und zum Separator 6 gefördert.

In den vorangegangenen Figuren 1 bis 3 ist der Übersichtlichkeit halber das Biomassesubstrat nicht eingezeichnet. Ebenfalls nicht eingezeichnet ist die an den Seitenwänden, den Böden 23 oder der Decke 24 anzuordnende Heizung, die dafür sorgt, dass eine Prozesstemperatur zwischen 25 und 35 Grad eingehalten wird.

In den Figuren 1 bis 3 kann nachvollzogen werden, dass die Verweildauer des Biomassesubstrates auf seinem Weg durch den erfindungsgemäßen Biomassetrockenfermenter hindurch durch die mittlere Drehzahl der insgesamt drei Förderschnecken bestimmt werden kann. Dabei sind nicht unbedingt drehzahlregelbare Antriebe erforderlich, weil die für eine bestimmte Verweildauer erforderliche, mittlere Drehzahl auch durch intermittierenden Betrieb, also ein regelmäßiges Aus- und Einschalten der Antriebe erreicht werden kann.

In den Figuren 1 bis 3 ist der Übersichtlichkeit halber ebenfalls nicht eingezeichnet, dass ein Teil des Perkolates 7 wieder zur ersten Förderschnecke 3 und/oder direkt in den Fermenterbehälter 2 geleitet wird, um das darin befindendliche Biomassesubstrat zu "impfen". In der Praxis wird dieser Anteil meist bei etwa 20 % liegen. Die restlichen 80% stehen für die Weiterverarbeitung zu Methangas im zweiten Teil des Biogasprozesses zur Verfügung.

### Bezugszeichenliste

- 1: Verteilerschnecke im Fermenterbehälter 2
- 2: Fermenterbehälter
- 21: Eingang des Fermenterbehälters 2
- 22: Entleerungsseite des Fermenterbehälters 2
- 23: Boden des Fermenterbehälters 2
- 24: Decke des Fermenterbehälters 2
- 3: erste Förderschnecke, fördert Biosubstrat in den Behälter 2
- 31: erster, gasdichter Abschieber zwischen Förderschnecke 3 und Eingang 21 des Fermenterbehälters 2
- 4: zweite Förderschnecke, entnimmt Biosubstrat aus Behälter 2
- 41: zweiter, gasdichter Abschieber zwischen Förderschnecke 4 und Entleerungsseite 22
- 5: Paddelrührwerk in Fermenterbehälter 2
- 6: Separator, von zweiter Förderschnecke befüllt.
- 7: Perkolat, durch Separator 5 aus vergorenem Biomassesubstrat abgetrennt
- 8: Feststoffe, durch Separator 5 aus vergorenem Biomassesubstrat abgetrennt

## Patentansprüche

1. Biomassetrockenfermenter mit kontinuierlicher Beschickung von Biomassesubstrat und kontinuierlicher Entnahme des vergorenem Biomassesubstrates, bestehend aus
- einem Fermenterbehälter 2, aus einem Separator 6 zur Trennung des vergorenen Biomassesubstrates in ein Perkolat 7 und in Feststoffe 8,
- einen Paddelrührwerk 5, das in der Mitte des Fermenterbehälters 2 horizontal liegend angebracht ist
**dadurch gekennzeichnet, dass**
- der Fementerbehälter 2 gasdicht ist, am Eingang 21 des Fermenterbehälters 2
- eine erste Förderschnecke 3 mit einem ersten gasdichten Abschieber 31 angeschlossen ist, mittels derer das Biomassesubstrat von oben in den Fermenterbehälter 2 einbringbar ist und
- einer zweiten Fördereinrichtung mit einem zweiten gasdichten Abschieber 41, über den das vergorene Substrat unten an der Entleerungsseite 22 des Fermenterbehälters 2 entnehmbar ist,
- der Boden 23 des Fermenterbehälters 2 von der Befüllseite zur gegenüberliegenden Entleerungsseite 22 hin sowie von den beiden angrenzenden Seiten zur Mitte hin abfällt und
- die zweite Fördereinrichtung eine Förderschnecke 4 ist,
- die in der Nähe des tiefsten Punktes des Bodens 23 angeordnet ist und mit dem Separator 6 verbunden ist.

2. Fermenter nach Anspruch 1, **dadurch gekennzeichnet, dass** der Boden 23 ein Gefälle zwischen 1 Grad und 60 Grad aufweist.

3. Fermenter nach einem der vorhergehenden Ansprüche, **dadurch gekennzeichnet, dass** der Boden 23 des Fermenterbehalters 2 aus drei zur Mitte hin abfallenden, aneinander grenzenden Ebenen besteht, die eine V-förmige Rille bilden, in die die zweite Förderschnecke 4 eingebaut ist.

4. Fermenter nach einem der vorhergehenden Ansprüche, **dadurch gekennzeichnet, dass** der Boden 23 und/oder die Seitenwände des Fermenterbehälters mit Edelstahl verkleidet sind.

5. Fermenter nach einem der vorhergehenden Ansprüche, **dadurch gekennzeichnet, dass** das Paddelrührwerk 5 auf den Außenseiten kürzere und in der Mitte längere Paddel aufweist.

6. Fermenter nach einem der vorhergehenden Ansprüche, **dadurch gekennzeichnet, dass** das in den Fermenterbehälter 2 eingebrachte Biomassesubstrat mittels einer Verteilerschnecke 1 auf die ganze Breite des Fermenterbehälters 2 verteilt wird.

7. Fermenter nach einem der vorhergehenden Ansprüche, **dadurch gekennzeichnet, dass** über eine Zuleitung die Beimischung von Perkolat oder Gülle möglich ist.

8. Fermenter nach einem der vorhergehenden Ansprüche, **dadurch gekennzeichnet, dass** die Zuleitung an der ersten Förderschnecke 3 oder in den Fermenterbehälter 2 oder an der Verteilerschnecke 1 mündet.

9. Fermenter nach einem der vorhergehenden Ansprüche, **dadurch gekennzeichnet, dass** eine Heizung
- an den Seiten und/oder
- am Boden 23 und /oder
- an der Decke 24
des Fermenterbehälters 2 angebracht ist.

10. Fermenter nach einem der vorhergehenden Ansprüche, **dadurch gekennzeichnet, dass** die Decke 24 des Fermenterbehälters 2
- aus einer gasdichten Plane oder
- aus dem gleichen Betonmaterial oder dem gleichen Eisenmaterial
wie die Seitenwände
besteht.

11. Fermenter nach einem der vorhergehenden Ansprüche, **dadurch gekennzeichnet, dass** an der Decke 24
- eine gasdichte Einstiegsluke und/oder
- ein Überdruckventil
angebracht ist.

12. Fermenter nach einem der vorhergehenden Ansprüche, **dadurch**
**gekennzeichnet, dass** in die Seiten gasdichte Kontrollöffnungen eingebracht sind.

13. Verfahren zur Erzeugung von Perkolat 7 aus Biomassesubstrat, das in den Eingang 21 des Fermenterbehälters 2 eines Biomassetrockenfermenters nach einem der Ansprüche 1-12 eingebracht wird, **dadurch gekennzeichnet, dass** das Biomassesubstrat in etwa kontinuierlich eintreffenden Portionen oder in einem etwa kontinuierlichen Strom in den Eingang 21 des Fermenterbehälters 2 eingebracht wird und mit Perkolat 7 und/oder Gülle oder einem anderen Material geimpft wird, das zur Einleitung des Versäuerungsprozesses geeignete Mikroben enthält und dann kontinuierlich innerhalb des Fermenterbehälters 2 weiter bewegt und dabei zu Perkolat 7 umgewandelt und das Perkolat 7 auf der Entleerungsseite 22 des Fermenterbehälters 2 entnehmbar ist.

## Claims

1. Dry biomass fermenter with continuous feeding of biomass substrate and continuous removal of the fermented biomass substrate, comprising
- a fermenter vessel 2, which comprises a separator 6 for separating the fermented biomass substrate into a percolate 7 and solids 8,
- a paddle agitator 5, which is mounted horizontally in the centre of
the fermenter vessel 2,
**characterised in that**
- the fermenter vessel 2 is gas tight, at the inlet 21 of the fermenter vessel 2
- a first conveying screw 3 which is connected to a first gastight pusher 31, by means of which the biomass substrate can be introduced from above into the fermenter vessel 2, and
- a second conveying device comprising a second gas-tight pusher 41, via which the fermented substrate can be removed at the bottom at the discharge side 22 of the fermenter vessel 2,
- the base 23 of the fermenter vessel 2 falls from the filling side towards the opposite discharge side 22 and from the two adjacent sides towards the centre, and
- the second conveyor device is a conveying screw 4,
- which is disposed in the vicinity of the lowest point of the base 23 and is connected to the separator 6.

2. Fermenter according to claim 1, **characterised in that** the base 23 has a gradient of between 1 degree and 60 degrees.

3. Fermenter according to one of the preceding claims, **characterised in that** the base 23 of the fermenter vessel 2 consists of three levels which are adjacent to one another and fall towards the centre, and form a V-shaped groove, in which the second conveyor screw 4 is installed.

4. Fermenter according to one of the preceding claims, **characterised in that** the base 23 and/or the side walls of the fermenter vessel are lined with stainless steel.

5. Fermenter according to one of the preceding claims, **characterised in that** the paddle agitator 5 has shorter paddles on the outer sides and longer paddles in the centre.

6. Fermenter according to one of the preceding claims, **characterised in that** the biomass substrate introduced into the fermenter vessel 2 is distributed by means of a distribution screw 1 over the entire width of the fermenter vessel 2.

7. Fermenter according to one of the preceding claims, **characterised in that** the admixture of percolate or slurry is possible via a feed line.

8. Fermenter according to one of the preceding claims, **characterised in that** the feed line opens on the first conveyor screw 3 or into the fermenter vessel 2, or on the distribution screw 1.

9. Fermenter according to one of the preceding claims, **characterised in that** a heater
- is mounted on the sides and/or
- on the base 23 and/or
- on the ceiling 24
of the fermenter vessel 2.

10. Fermenter according to one of the preceding claims, **characterised in that** the ceiling 24 of the fermenter vessel 2
- consists of a gastight tarpaulin
- of the same concrete material or the same ferrous material as the side walls

11. Fermenter according to one of the preceding claims, **characterised in that** on the ceiling 24, there is mounted
- a gastight access hatch and/or
- a pressure relief
valve.

12. Fermenter according to one of the preceding claims, **characterised in that** gastight control openings are introduced into the sides..

13. Method for generating percolate 7 from biomass substrate, which is introduced into the inlet 21 of the fermenter vessel 2 of a dry biomass fermenter according to one of claims 1-12, **characterised in that** the biomass substrate is introduced in approximately continuously arriving portions or in an approximately continuous stream into the inlet 21 of the fermenter vessel 2, and is inoculated with percolate 7 and/or slurry or another material, which contains suitable microbes for initiating the acidification process, and then moves further continuously within the fermenter vessel 2 and in the process is converted into percolate 7, and the percolate 7 is removed at the discharge side 22 of the fermenter vessel 2.

## Revendications

1. Fermenteur à sec de biomasse avec une alimentation continue de substrat de biomasse et prélèvement continu du substrat de biomasse fermenté, consistant en
- un réservoir du fermenteur 2, un séparateur 6 destiné à séparer le substrat de biomasse fermenté en un percolat 7 et en matières solides 8,
- un mélangeur à palettes 5 qui est disposé couché à
l'horizontale au milieu du réservoir du fermenteur 2 **caractérisé par le fait**
- **que** le réservoir du fermenteur 2 est étanche au gaz, à l'entrée 21 du réservoir du fermenteur,
- **qu'**une première vis d'extraction 3 est raccordée avec un premier expulseur 31 au moyen duquel le substrat de biomasse peut être amené depuis le haut dans le réservoir du fermenteur 2 et
- **qu'**il existe un deuxième dispositif de convoyage avec un second expulseur 41 étanche au gaz à l'aide duquel le substrat fermenté peut être prélevé en bas du côté de la vidange 22 du réservoir du fermenteur 2,
- le fond 23 du réservoir du fermenteur 2 retombant depuis le côté du remplissage vers le côté de la vidange 22 ainsi que depuis les deux côtés avoisinants vers le milieu et
- **que** le deuxième dispositif de convoyage est une vis d'extraction 4
■ qui est disposée à proximité du point le plus bas du fond 23 et est reliée au séparateur 6.

2. Fermenteur à sec de biomasse selon la revendication 1, **caractérisé par le fait que** le fond 23 présente une déclivité comprise entre 1 degré et 60 degrés.

3. Fermenteur à sec de biomasse selon une des revendications précédentes, **caractérisé par le fait que** le fond 23 du réservoir du fermenteur 2 consiste en trois niveaux retombants, voisins les uns des autres, qui forment une rainure en forme de V dans laquelle est intégrée une seconde vis d'extraction 4.

4. Fermenteur à sec de biomasse selon une des revendications précédentes, **caractérisé par le fait que** le fond 23 et/ou les parois latérales du réservoir du fermenteur sont habillées d'acier inox.

5. Fermenteur à sec de biomasse selon une des revendications précédentes, **caractérisé par le fait que** le mélangeur à palettes 5 présente des palettes plus courtes sur ses faces extérieures et des palettes plus longues au milieu.

6. Fermenteur à sec de biomasse selon une des revendications précédentes, **caractérisé par le fait que** le substrat de biomasse apporté dans le réservoir du fermenteur 2 est réparti sur toute la largeur du réservoir du fermenteur 2 à l'aide d'une vis de répartition 1.

7. Fermenteur à sec de biomasse selon une des revendications précédentes, **caractérisé par le fait qu'**il est possible d'adjoindre du percolat ou du lisier via une conduite d'alimentation.

8. Fermenteur à sec de biomasse selon une des revendications précédentes, **caractérisé par le fait que** la conduite d'alimentation débouche sur la première vis d'extraction 3 ou dans le réservoir du fermenteur 2 ou sur la vis de répartition 1

9. Fermenteur à sec de biomasse selon une des revendications précédentes, **caractérisé par le fait qu'**un chauffage est disposé
- sur les côtés et/ou
- sur le fond 23
- sur le plafond 24.

10. Fermenteur à sec de biomasse selon une des revendications précédentes, **caractérisé par le fait que** le plafond 24 du réservoir du fermenteur 2 consiste
- en une bâche étanche au gaz ou
- en le même matériau en béton ou le même matériau ferreux que les parois latérales.

11. Fermenteur à sec de biomasse selon une des revendications précédentes, **caractérisé par le fait**
- **qu'**une trappe d'accès étanche au gaz et/ou
- une soupape de surpression
est disposée sur le plafond 24.

12. Fermenteur à sec de biomasse selon une des revendications précédentes, **caractérisé par le fait que** des ouvertures de contrôles étanches au gaz sont disposées dans les côtés.

13. Procédé destiné à générer du percolat 7 de substrat de biomasse, qui est disposé à l'entrée 21 du réservoir du fermenteur 2 d'un fermenteur à sec de biomasse selon une des revendications 1 - 12, **caractérisé par le fait que** le substrat de biomasse est apporté dans des portions qui arrivent dans un flux à peu près continu dans l'entrée 21 du réservoir du fermenteur 2 et se voit injecter du percolat 7 et/ou du lisier ou un autre matériau qui contient des microbes convenant pour initier le processus d'acidification et le déplace ensuite de façon continue à l'intérieur du réservoir du fermenteur 2 et le convertit dans ce cadre en percolat 7, le percolat 7 pouvant être prélevé du côté de la vidange 22 du réservoir du fermenteur 2.
